(19) **Europäisches Patentamt / European Patent Office / Office européen des brevets**

(11) **EP 2 820 050 B1**

(12) **FASCICULE DE BREVET EUROPEEN**

(45) Date de publication et mention
de la délivrance du brevet:
**29.04.2026 Bulletin 2026/18**

(21) Numéro de dépôt: **13712862.5**

(22) Date de dépôt: **27.02.2013**

(51) Classification Internationale des Brevets (IPC):
*C08B 30/18* (2006.01)  *A61K 31/718* (2006.01)
*C12P 19/04* (2006.01)  *C12P 19/20* (2006.01)

(52) Classification Coopérative des Brevets (CPC):
(C-Sets disponibles)
**C08B 30/18; A23L 5/00; A23L 29/30; A23L 29/35;
A23L 33/10; A23L 33/125; A23L 33/20; A61P 3/10;
C12P 19/20;** A23V 2002/00          (Cont.)

(86) Numéro de dépôt international:
**PCT/FR2013/050407**

(87) Numéro de publication internationale:
**WO 2013/128121 (06.09.2013 Gazette 2013/36)**

(54) **MALTODEXTRINES HYPERBRANCHEES HYPO-GLYCEMIANTES**

HYPERVERZWEIGTE HYPOGLYKÄMISCHE MALTODEXTRINE

HYPOGLYCEMIC HYPER-BRANCHED MALTODEXTRINS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **28.02.2012 FR 1251810**

(43) Date de publication de la demande:
**07.01.2015 Bulletin 2015/02**

(73) Titulaire: **Roquette Frères
62136 Lestrem (FR)**

(72) Inventeurs:
• **BUREAU, Stéphanie
F-62400 Essars (FR)**
• **GUERIN-DEREMAUX, Laëtitia
F-59850 Nieppe (FR)**
• **PORA, Bernard
Wuhan
Hubei 430056 (CN)**

(74) Mandataire: **Roquette IP International
Roquette Frères
1, rue de la Haute Loge
62136 Lestrem (FR)**

(56) Documents cités:
**EP-A1- 0 535 627     EP-A1- 0 540 421
EP-A1- 1 006 128     EP-A2- 0 368 451
WO-A1-2009/075564     JP-A- H04 135 495
JP-A- H04 237 497**

• **"Starch: Chemistry and Technology", 1984,
ACADEMIC PRESS, pages: 153 - 154**

**EP 2 820 050 B1**

(52) Classification Coopérative des Brevets (CPC):
(Cont.)

C-Sets
A23V 2002/00, A23V 2200/32, A23V 2200/328,
A23V 2250/5114

2

**Description**

L'invention concerne de nouvelles maltodextrines hyperbranchées de faible poids moléculaire, i.e. présentant un dextrose équivalent (DE) compris entre 8 et 15 et un poids moléculaire ou Mw compris entre 1700 et 3000 daltons, caractérisées par leur teneur particulière en liaisons glucosidiques 1 → 6, leur teneur en fibres indigestibles solubles, et surtout par leurs remarquables propriétés hypoglycémiantes.

Plus particulièrement, ces nouvelles maltodextrines hyperbranchées présentent une teneur en liaisons glucosidiques 1 → 6 comprise entre 30 et 45 %, une teneur en fibres indigestibles solubles comprise entre 75 et 100 % (selon la méthode AOAC N°2001-03) et de remarquables propriétés hypoglycémiantes, qu'elles traduisent *in vitro* comme *in situ,* par un effet protecteur vis-à-vis de la digestion de maltodextrines standard.

Au sens de l'invention, les « maltodextrines standard » se définissent comme des mélanges purifiés et concentrés de glucose et de polymères de glucose essentiellement liés en α-1,4 avec seulement 4 à 5 % de liaisons glucosidiques α-1,6, de poids moléculaires variés, complètement solubles dans l'eau et à faible pouvoir réducteur.

Ces maltodextrines standard sont classiquement produites par hydrolyse acide ou enzymatique d'amidon de céréales ou de tubercules. La classification des maltodextrines standard repose principalement sur la mesure de leur pouvoir réducteur, exprimé classiquement par la notion de Dextrose Equivalent ou D.E. Sur ce point particulier, la définition des maltodextrines reprise dans les *Monograph Specifications du Food Chemical Codex* précise que la valeur de D.E. ne doit pas excéder 20.

Au sens de l'invention, l'« effet protecteur » qu'expriment les maltodextrines hyperbranchées de l'invention en regard des maltodextrines standard se traduit par leur capacité, lorsque mélangées avec des maltodextrines standard :

-    *in vitro,* à réduire de 80 à 90 % l'hydrolyse par l'α-amylase desdites maltodextrines standard ;
-    *in situ,* dans l'intestin, à réduire de 30 à 45 % l'activité digestive intestinale de ces maltodextrines standard.

En agissant sur la digestion et l'absorption des hydrates de carbone, e.g. les maltodextrines standard, les maltodextrines hyperbranchées de l'invention ralentissent et réduisent donc l'augmentation de la glycémie consécutive à un repas (post prandiale), ainsi que la sécrétion d'insuline.

Cette action est donc susceptible d'aider les diabétiques à améliorer le contrôle de leur glycémie.

L'invention vise ainsi des compositions comprenant de telles maltodextrines hyperbranchés utilisables dans de nombreuses applications industrielles, notamment dans les industries alimentaires et pharmaceutiques.

L'invention concerne enfin un procédé de fabrication desdites maltodextrines hyperbranchées.

Depuis quelque temps, un intérêt considérable s'est porté à la définition de régimes alimentaires appropriés riches en fibres. Il est en effet reconnu que l'apport de fibres dans l'alimentation a un effet bénéfique sur la santé.

Les fibres alimentaires sont souvent classées en fonction de leur solubilité, en fibres insolubles et solubles.

Ces deux types de fibres sont présents en proportions variables dans les produits alimentaires contenant des fibres. L'avoine, l'orge, les fruits, les légumes frais et les légumes secs (haricots, lentilles, pois chiches) constituent de bonnes sources de fibres solubles, tandis que les céréales complètes et le pain complet sont riches en fibres insolubles.

Les fibres insolubles, comme la cellulose, les amidons résistants, les fibres de maïs (drêche) ou de soja, ont un rôle essentiellement mécanique dans le tractus gastro-intestinal.

Elles ne sont que très peu fermentées par la flore intestinale et contribuent à la réduction du temps de transit intestinal par effet de lest.

Les fibres insolubles contribuent ainsi à prévenir la constipation en augmentant le poids des selles et en réduisant la durée du transit intestinal.

Les fibres solubles, comme la pectine et l'inuline, non digestibles par les enzymes intestinales de l'homme, sont fermentées par la flore bactérienne intestinale. Cette fermentation libère des acides gras à courte chaîne dans le côlon, qui ont pour effet de diminuer le pH de celui-ci et par voie de conséquence de limiter le développement de bactéries pathogènes et de stimuler le développement des bactéries bénéfiques.

Les acides gras à chaîne courte constituent également une source importante d'énergie pour les cellules du côlon et inhibent la croissance et la prolifération des cellules cancéreuses de l'intestin.

Les mécanismes invoqués pour expliquer les effets bénéfiques des fibres alimentaires sur les métabolismes glucidique et lipidique sont multiples ; ils ne s'excluent pas les uns les autres.

L'effet immédiat des fibres solubles sur la glycémie postprandiale a imposé une explication "mécanique" à l'action des fibres alimentaires :

-    allongement du temps de vidange gastrique par augmentation de la viscosité du bol alimentaire ;
-    effet de dilution et de barrière à l'action des enzymes digestives sur les aliments dans l'intestin grêle ;
-    augmentation de la couche visqueuse qui tapisse l'intestin grêle, ralentissant aussi le temps d'absorption des nutriments ;

- étalement de l'absorption des nutriments par augmentation du temps, de la bouche à l'intestin.

Les premiers travaux qui datent de la fin des années 70, montraient que le fait de donner à des sujets diabétiques un régime riche en hydrates de carbone et en fibres améliorait l'équilibre glycémique et diminuait leurs besoins en insuline ce qui allait à l'encontre des tendances diététiques et nutritionnelles de l'époque.

Les effets à court terme des fibres alimentaires sur la glycémie et l'insulinémie postprandiales sont largement documentés et cohérents : elles affectent l'élévation glycémique postprandiale aussi bien chez les sujets diabétiques insulinotraités, non insulinodépendants, intolérants au glucose que chez les sujets sains. Cet effet est d'autant plus net qu'il s'agit de fibres solubles.

Il résulte donc des nombreuses études qu'il existe une relation entre les sucres complexes (polysaccharides, amidon) et la bonne physiologie du côlon.

L'impact de ces sucres complexes sur le contrôle de la glycémie a été étudié via le devenir des amidons résistants, non digérés dans l'intestin grêle, présentant ainsi un grand intérêt pour la santé du côlon.

Les cibles de leurs effets fonctionnels sont normalement la flore colique qui les fermente et pour laquelle ils servent de substrats spécifiques et sélectifs, la physiologie gastro-intestinale et en particulier les fonctions assurées par le gros intestin, le système immunitaire, la biodisponibilité des minéraux et le métabolisme des lipides.

On retient donc que les fibres solubles :

- ralentissent la vidange gastrique ;
- procurent une satiété précoce ;
- diminuent la vitesse d'absorption des glucides (et aussi des lipides) dans l'intestin grêle.

Les composés classiquement classés dans les fibres solubles sont les fructooligosaccharides et les transgalacto-oligosaccharides, mais également le lactulose, les isomaltooligosaccharides, les oligosaccharides extraits du soja, les xylooligosaccharides...

Par exemple les fructooligosaccharides (FOS) sont des polymères d'unités fructose à courtes chaînes qui ne sont pas hydrolysés dans l'intestin grêle chez l'Homme, mais sont dégradés par la flore résidente du côlon.

Les FOS induisent principalement la croissance des lactobacilles et des bifidobactéries endogènes de l'intestin chez l'Homme et les animaux.

A côté de ces composés majoritairement extraits des plantes, existent des molécules dérivées de l'amidon ou de ses produits d'hydrolyse partielle ou totale.

Le polydextrose est par exemple synthétisé par polymérisation aléatoire du glucose en présence de sorbitol et d'un catalyseur acide adéquat (tel l'acide citrique) et à haute température.

Le polydextrose est largement utilisé dans l'alimentation comme agent de charge et comme ingrédient à faible caloricité. Le polydextrose n'est pas digéré ni absorbé dans l'intestin grêle et une partie importante est retrouvée dans les fèces.

Le polydextrose et souvent associé avec les FOS, car il favoriserait ainsi la consommation d'acide lactique par des flores spécifiques, contrebalançant sa surproduction induite par les FOS.

Mais il est également connu dans l'état de la technique un certain nombre de technologies qui ont été développées afin de traiter l'amidon pour lui conférer des propriétés de fibres alimentaires, et obtenir ainsi des amidons résistants (ENGLYST et CUMMINGS dans American Journal of Clinical Nutrition en 1987, volume 45 pp 423-431).

Il est ainsi classiquement procédé au traitement d'un amidon par un acide alimentaire à haute température. Ce traitement thermique va alors générer des dérivés d'amidon de type pyrodextrines, dextrines blanches ou jaunes, en fonction de la dose d'acide, de la teneur en eau des amidons, et des intervalles de température mis en œuvre, ces dérivés d'amidon étant résistants à la digestion et à l'absorption dans l'intestin grêle chez l'Homme.

En effet, si les amidons et maltodextrines digestibles standard ne possèdent que des liaisons glucosidiques de type $\alpha$,-1,4 et $\alpha$,-1,6, le traitement thermique en conditions acides et en faible teneur en eau va produire des liaisons atypiques de type 1,2 et 1,3 (en anoméries alpha ou beta) qui ne sont pas hydrolysées par les enzymes de digestion humaines.

Ces traitements physiques sont souvent complétés par des traitements enzymatiques pour renforcer le caractère de fibres alimentaires des dérivés d'amidon ainsi obtenus.

Dans les brevets EP 368.451 et US 5.264.568, par exemple, est ainsi décrit un procédé de préparation de pyrodextrines, dont on renforce les caractéristiques de fibres alimentaire par action d'une $\alpha$-amylase ou de plusieurs $\alpha$-amylases successivement sur une dextrine ou sur une pyrodextrine en solution à haute température.

Dans le brevet EP 530.111, sont décrites des dextrines indigestibles obtenues par extrusion d'un amidon de maïs acidifié déshydraté dans des conditions particulières. Ce traitement peut être complété par l'action d'une $\alpha$-amylase thermorésistante.

Le document JP04237497 décrit à l'exemple 2 un procédé de préparation d'une dextrine indigestible comprenant la fourniture d'une dextrine grillée, suivie d'un traitement de cette dextrine grillée par une glucoamylase, suivie d'une étape de séparation du glucose de la dextrine grillée traitée, suivie d'une étape de récupération de la dextrine indigestible.

Le document JP 04136495 décrit aux exemples un procédé de préparation de polysaccharides faiblement digestibles comprenant la fourniture d'un amidon présentant une humidité de 12%, suivie d'une étape de grillage de cet amidon, puis d'un traitement de cet amidon grillé par une glucoamylase, suivie d'une étape de séparation du glucose de la dextrine grillée traitée, suivie d'une étape de fractionnement pour obtenir les polysaccharides faiblement digestibles.

Le document EP540421 décrit à la revendication 1 une dextrine indigestible fabriquée par un procédé comprenant la fabrication d'une pyrodextrine par réaction d'acide chlorhydrique sur une fécule de pomme de terre suivie d'une étape d'hydrolyse par une alpha-amylase et une glucoamylase, puis une étape de suppression au moins partielle du glucose pour former la dextrine indigestible.

Le document EP535627 à la revendication 1 une dextrine indigestible fabriquée par un procédé comprenant la fabrication d'une pyrodextrine par réaction d'acide chlorhydrique sur un amidon de maïs suivie d'une étape d'hydrolyse par une alpha-amylase et une glucoamylase, puis une étape de suppression au moins partielle du glucose pour former la dextrine indigestible.

La société Demanderesse a elle-même également décrit dans sa demande de brevet EP 1.006.128 des « maltodextrines branchées » présentant entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole.

Ces maltodextrines branchées présentent surtout un caractère d'indigestibilité qui a pour conséquence de diminuer leur pouvoir calorique, en empêchant leur assimilation au niveau de l'intestin grêle, et elles constituent ainsi une source de fibres indigestibles.

A noter que des fibres peuvent être dosées selon différentes méthodes AOAC. On peut citer à titre d'exemple, les méthodes AOAC 997.08 et 999.03 pour les fructanes, les FOS et l'inuline, la méthode AOAC 2000.11 pour le polydextrose, la méthode AOAC 2001.03 pour le dosage des fibres contenues dans les maltodextrines branchées ou la méthode AOAC 2001.02 pour les GOS ainsi que les oligosaccharides solubles d'origine oléagineuse ou protéagineuse.

Tous ces sucres complexes impactent la glycémie par leur propre caractère indigestible, mais fermentescible par les bactéries bénéfiques du côlon, contribuant ainsi à l'intégrité de la barrière intestinale.

L'homme du métier du domaine considéré utilise donc cette gamme de produits pour leurs propriétés intrinsèques.

Ce caractère indigestible a ainsi été développé par exemple dans le brevet EP 443.789 comme moyen permettant d'offrir une composition alimentaire régulant la glycémie en abaissant la sécrétion d'insuline sans impacter le niveau de glucose sanguin.

Mais peu d'études ont été entreprises pour proposer des composés de faible poids moléculaire, présentant des propriétés intrinsèquement indigestibles, susceptibles d'agir en plus spécifiquement comment agents barrières de l'action des enzymes digestives sur les aliments, même si, comme dans les travaux de LIVESEY, G et TAGAMI, H publié dans Am. J. Clin. Nutr., 2009, 89, 114-25, un des 6 mécanismes évoqués en toute généralité comme susceptible d'expliquer l'effet des maltodextrines résistantes sur la glycémie postprandiale puisse être une inhibition enzymatique.

De tout ce qui précède, il résulte qu'il n'existe pas, à la connaissance de la société Demanderesse, de polysaccharides de faible poids moléculaire et hautement branchés présentant de tels effets hypoglycémiants par réduction de la digestion des hydrates de carbone.

La société Demanderesse a eu le mérite d'imaginer et d'élaborer, au prix de nombreuses recherches, de nouvelles maltodextrines hyperbranchées présentant spécifiquement cet effet barrière.

Les maltodextrines hyperbranchées conformes à l'invention constituent une nouvelle famille au sens où elle est bien différente de celles de l'état de la technique, y compris des autres maltodextrines branchées de faible poids moléculaire que la société Demanderesse a déjà proposées et décrites dans ses propres demandes de brevet antérieures.

Par maltodextrines branchées, on entend au sens de l'invention les maltodextrines décrites dans le brevet EP 1.006.128 dont la société Demanderesse est titulaire.

Plus particulièrement, les maltodextrines hyperbranchées de l'invention présentent un dextrose équivalent (DE) compris entre au moins 8 et au plus 15 et un poids moléculaire ou Mw compris entre au moins 1700 et au plus 3000 daltons.

Elles sont surtout caractérisées par :

- une teneur en liaisons glucosidiques $1 \rightarrow 6$ comprise entre au moins 30 et au plus 45 %,
- une teneur en fibres indigestibles solubles, déterminée selon la méthode AOAC N°2001-03, comprise entre au moins 75 et au plus 100 %, et
- une capacité hypoglycémiante, exprimée selon un test A, se traduisant :

    - *in vitro,* par la réduction de 80 à 90 % de l'hydrolyse par l'$\alpha$-amylase de maltodextrines produites par hydrolyse acide ou enzymatique d'amidon de céréales ou de tubercules, dites « maltodextrines standard »,

- *in situ,* par la réduction de 30 à 45 % de l'activité digestive intestinale de maltodextrines standard.

Une première famille de produits conformes à l'invention est constituée par des maltodextrines hyperbranchées présentant un DE compris entre au moins 8 et au plus 12 et un Mw compris entre au moins 2500 et au plus 3000 daltons, caractérisée par :

- une teneur en liaisons glucosidiques 1 → 6 comprise entre au moins 30 et au plus 35 %,
- une teneur en fibres indigestibles solubles, déterminée selon la méthode AOAC N°2001-03, comprise entre au moins 75 et au plus 80 %.

Une deuxième famille de produits conformes à l'invention est constituée par des maltodextrines hyperbranchées présentant un DE compris entre au moins 12 et au plus 15 et un Mw compris entre au moins 1700 et au plus 2500 daltons, caractérisée par :

- une teneur en liaisons glucosidiques 1 → 6 comprise entre au moins 35 % et au plus 45 %,
- une teneur en fibres indigestibles solubles, déterminée selon la méthode AOAC N°2001-03, comprise entre au moins 80 et au plus 100 %.

Les maltodextrines hyperbranchées conformes à l'invention sont tout d'abord caractérisées par leur DE et par leur poids moléculaire.

Comme indiqué ci-avant, la société Demanderesse a développé et rapporté dans sa demande de brevet EP 1.006.128 des « maltodextrines branchées » présentant entre 15 et 35 % de liaisons glucosidiques 1-6, une teneur en sucres réducteurs inférieure à 20 %, un indice de polymolécularité inférieur à 5 et une masse moléculaire moyenne en nombre Mn au plus égale à 4500 g/mole.

Les maltodextrines hyperbranchées selon l'invention, par leur DE et leur poids moléculaire, s'apparentent à cette famille de maltodextrines branchées.

Les paramètres analytiques du DE et du poids moléculaire (ou Mw) sont déterminées par tout méthode connue par ailleurs de l'homme du métier :

- la méthode de détermination du Dextrose Equivalent est par exemple la méthode à titrage constant de LANE-EYNON (1923, Determination of reducing sugars by means of Fehling's solution with methylene blue as internal indicator. J. Soc. Chem. Ind. Trans. 32-36*) ;*
- les valeurs de Mw sont mesurées par chromatographie d'exclusion stérique, fondée sur la rétention sélective des molécules du soluté en fonction de leur taille, en raison de leur pénétration ou non dans les pores de la phase stationnaire.

Les maltodextrines hyperbranchées de l'invention présentent ainsi un DE limité à une valeur comprise entre 8 et 15, pour un poids moléculaire faible, compris entre 1700 et 3000 daltons.

Par ailleurs, et c'est là où les maltodextrines hyperbranchées selon l'invention se différencient des maltodextrines branchées de l'EP 1.006.128, elles présentent :

- une teneur en liaisons glucosidiques 1 → 6 globalement plus élevée, comprise entre 30 et 45 %,
- une teneur en fibres indigestibles solubles importante, comprise entre 75 et 100 % (selon la méthode AOAC N °2001-03) et surtout
- de remarquables propriétés hypoglycémiantes.

La détermination de la teneur en liaisons glucosidiques 1 → 2, 1 → 3, 1 → 4 et 1 → 6 est réalisée selon la technique classique de méthylation décrite dans HAKOMORI, S. 1964, J. Biol. Chem, 55, 205.

Quant aux propriétés hypoglycémiantes, elles sont déterminées par la mise en œuvre d'un test de digestion enzymatique *in vitro* et *in situ,* permettant de mesurer la capacité des maltodextrines hyperbranchées de l'invention à réduire la digestion de maltodextrines standard.

Pour sa composante « *in vitro* », ce test consiste à mesurer au cours du temps la quantité de sucres réducteurs libérés par action de l'$\alpha$-amylase pancréatique de porc sur les maltodextrines standard en présence des maltodextrines hyperbranchées de l'invention.

Le mode opératoire est le suivant :

- dans un bécher de 250 ml, peser exactement 50,0 g de maltodextrines standard du type de celles commercialisées par la société Demanderesse sous le nom de marque GLUCIDEX 6,
- peser exactement 5,0 g de maltodextrines hyperbranchées à tester et les introduire dans le bécher,
- solubiliser avec 150 ml d'eau déminéralisée,
- rectifier le pH à la valeur de 5, si nécessaire,
- transvaser dans une fiole jaugée de 250 ml, rincer le bécher avec un peu d'eau et ajuster la fiole à 250 ml avec de l'eau déminéralisée,
- transvaser dans un Erlenmeyer de 500 ml,
- placer dans un incubateur à 37°C,
- ajouter 25 mg d'$\alpha$-amylase pancréatique de porc commercialisée par SIGMA sous la référence A3176 (Type VI-B, $\geq$ 10 unités/mg solide)
- prélever 50 ml de solution à 3 heures, 6 heures et 24 heures de réaction, et inactiver 10 minutes au bain marie à 85°C,
- mesurer la teneur en sucres réducteurs selon la méthode de Gabriel Bertrand ("Bulletin des sciences pharmacologiques", t.14, n° 1, janv. 1907)

Les mesures sont également effectuées sur le témoin (digestion de la GLUCIDEX® 6 seule à l'$\alpha$-amylase) et les résultats de ce test in vitro sont exprimés en % d'activité $\alpha$-amylasique versus le témoin.

Comme il sera exemplifié ci-après, les maltodextrines hyperbranchées de l'invention parviennent ainsi à réduire de 80 à 90 % l'hydrolyse de la GLUCIDEX® 6 par l'$\alpha$-amylase pancréatique.

A titre comparatif, les maltodextrines branchées développées par la société Demanderesse dans son brevet EP 1.006.128 ne parviennent qu'à réduire de 30 à 50 % l'hydrolyse de la GLUCIDEX® 6 par l'$\alpha$-amylase pancréatique.

Pour sa composante « in situ », le test consiste à réaliser une perfusion intestinale continue chez le rat, de manière à calculer le pourcentage d'hydrolyse de maltodextrines standard.

Le mode opératoire est le suivant :

L'intestin grêle d'un rat OFA d'origine Sprague-Dawley, d'un poids d'environ 300-350 g préalablement anesthésié, est perfusé au niveau du duodénum et de l'iléon.

Un circuit fermé, dans lequel circule un courant constant est réalisé.

Le courant est assuré par une pompe péristaltique.

Une solution du produit à tester est injectée dans le circuit.

A cette solution est ajouté du polyéthylène-glycol (PEG) de masse moléculaire voisine de 4000. Celui-ci est utilisé comme marqueur des mouvements d'eau dans l'intestin.

Au cours des 2 heures de perfusion, les effluents intestinaux sont prélevés.

Après hydrolyse acide totale, la quantité de glucose dosée dans les effluents intestinaux, corrigée du ratio de PEG (avant et après perfusion) permet de calculer le pourcentage d'hydrolyse du produit testé.

Le protocole détaillé est le suivant :

- réaliser une solution tampon pH 7 RAL® à 4,68 g/l dans de l'eau physiologique (NaCl 0,15 M),
- réaliser une solution de PEG4000 à 1 % dans la solution tampon,
- réaliser une solution à perfuser du produit à tester à 10g/L dans la solution PEG4000 tamponnée,
- placer les animaux à jeun pendant 24 heures
- anesthésier l'animal sous isoflurane,
- effectuer une laparotomie,
- perfuser le duodénum et l'iléon (à environ 5 cm du cæcum) à l'aide du tuyau silicone ID 2mm,
- maintenir la température corporelle de l'animal sur une plaque chauffante réglée sur 37°C,
- introduire 30 ml de solution à perfuser dans ce circuit fermé,
- mettre en fonctionnement la pompe péristaltique, réglée sur 1 ml/min,
- réaliser des prélèvements à 30, 60 et 120 minutes, Les analyses sont ensuite effectuées de la façon suivante :
- réaliser le dosage du PEG sur la solution de perfusion initiale (Po) et sur l'effluent (Pt),
- réaliser le dosage du glucose dans l'effluent (GLt = Glucose Libre au temps t),
- réaliser une hydrolyse acide totale sur l'échantillon initial de produit et sur l'effluent,
- doser le glucose (GTo : Glucose Total au temps 0 - GTt : Glucose Total au temps t).

Les formules utilisées sont les suivantes :

- Glucose Branché (g/l) = GB = GTt - GLt
- Glucose branché corrigé des mouvements d'eau dans la lumière intestinale (GB'):

$$GB' = GB \times \frac{Po}{Pt}$$

-   Pourcentage d'hydrolyse du produit testé:

$$\% \text{ hydrolyse} = \frac{(GTo - GB')}{GTo} \times 100$$

Comme il sera exemplifié ci-après, les maltodextrines hyperbranchées de l'invention parviennent ainsi à réduire de 30 à 45 % l'hydrolyse de la GLUCIDEX® 6 en fin de perfusion intestinale.

Pour préparer les maltodextrines hyperbranchées conformes à l'invention, on réalise la succession des étapes suivantes :

a. on prépare un amidon acidifié déshydraté présentant une humidité inférieure à 5 %, de préférence inférieure ou égale à 4 %,

b. on traite l'amidon acidifié ainsi déshydraté dans un réacteur de type à couche mince à une température comprise entre 120 et 300°C, de préférence comprise entre 150 et 200°C,

c. on recueille, purifie et de préférence concentre les dérivés d'amidon branchés ainsi obtenus,

d. on effectue un fractionnement moléculaire desdits dérivés d'amidon branchés de manière à obtenir une fraction présentant :

i. un Mn compris entre 250 et 400 g/mole,
ii. un indice de polymolécularité compris entre 2 et 3,
iii. une teneur en sucres réducteurs comprise entre 20 et 30 %,
iv. un taux de liaisons glucosidiques $1 \rightarrow 6$ compris entre 30 et 35 %,

e. éventuellement, on traite la fraction de bas poids moléculaire ainsi obtenue par une amyloglucosidase,

f. on traite la solution ainsi obtenue sur colonne chromatographique de manière à exclure les oligomères de degré de polymérisation 1 et 2,

g. on récupère les maltodextrines hyperbranchées ainsi obtenues.

Pour les quatre premières étapes du procédé conforme à l'invention (étape a. à étape d.), étapes qui conduisent à la préparation de la fraction de bas poids moléculaire, toutes méthodes accessibles à l'homme du métier peuvent être employées ici.

Cependant, la société Demanderesse recommande d'utiliser celles décrites dans sa demande de brevet EP 1.006.128, étapes incorporées ici par référence (plus particulièrement celles de l'exemple 2 de l'EP 1.006.128 avant qu'il ne soit procédé à l'étape d'élimination du glucose par la glucose oxydase).

L'isolement de la fraction de bas poids moléculaire présentant :

i. un Mn compris entre 250 et 400 g/mole,
ii. un indice de polymolécularité compris entre 2 et 3,
iii. une teneur en sucres réducteurs comprise entre 20 et 30 %,
iv. un taux de liaisons glucosidiques $1 \rightarrow 6$ compris entre 30 et 35 %,

s'effectue alors au niveau du quatrième plateau de la colonne de chromatographie sur résine cationique macroporeuse de PUROLITE C 145 sous forme potassium, de granulométrie 250 - 350 $\mu$m, configurée en 6 plateaux.

La cinquième étape du procédé (étape e) conforme à l'invention, qui est facultative, consiste à traiter la fraction de bas poids moléculaire ainsi obtenue par une amyloglucosidase.

Ce traitement enzymatique a pour objectif d'hydrolyser majoritairement les structures linéaires du produit ainsi obtenu (liaison glucosidiques $\alpha$ $1 \rightarrow 4$) de manière à optimiser le contenu en liaisons glucosidiques indigestibles.

Comme il sera exemplifié, il est choisi un traitement avec une amyloglucosidase de type OPTIDEX L300A (de la société GENENCOR) à raison de 0,5 % sur sec, à un pH de 4,5 pendant de 5 à 10 h, de préférence pendant 8 heures.

La sixième étape du procédé (étape f) conforme à l'invention consiste à traiter la solution ainsi obtenue sur colonne chromatographique de manière à exclure les oligomères de degré de polymérisation 1 et 2.

Cette étape peut être réalisée par tout moyen connu de l'homme du métier, par exemple par chromatographie sur résine DIAION UBR 35 K sous forme sodique, commercialisée par la société MITSUBISHI.

Un rendement massique de 40 % permet, comme il sera exemplifié ci-après, de limiter la teneur en DP1 + DP2 à une valeur < 6 % sur sec, de préférence < 0,5 % sur sec.

La dernière étape du procédé conforme à l'invention consiste enfin à récupérer les maltodextrines hyperbranchées ainsi obtenues.

L'invention sera mieux comprise à l'aide des exemples qui suivent, lesquels se veulent illustratifs et non limitatifs.

**Exemple 1 : Préparation des maltodextrines hyperbranchées conformes à l'invention**

De l'amidon de blé est acidifié par de l'acide chlorhydrique à raison de 19,6 meq H+/kg sec, puis séché à une humidité résiduelle de 4 %.

Cette matière première est alors introduite dans un malaxeur BÜSS de type PR 46 maintenu à une température de 180°C, à un débit de 20 kg/h, avec un temps de séjour de 5 secondes.

On stoppe rapidement la réaction par pulvérisation d'eau froide à 15°C.

Après purification par filtration, décoloration sur résines adsorbantes et sur résines cationiques et anioniques, les dérivés d'amidon branchés ainsi obtenus sont ramenés à une matière sèche de 50 %.

Le produit obtenu est soumis à une chromatographie sur résine cationique forte macroporeuse de PUROLITE C 145 sous forme potassium, de granulométrie 250-350 $\mu$m, configurée en 6 plateaux de 200 litres, maintenus à 75°C.

Les débits d'alimentation du sirop de dérivés d'amidon branchés et de l'eau d'élution sont fixés à 60 1/h et 400 l/h, au niveau du premier et du troisième plateau, respectivement. Le choix des débits de sortie du deuxième et du quatrième plateau conditionne l'obtention des fractions de maltodextrines branchées de haut poids moléculaire et de bas poids moléculaire.

On fixe le débit en sortie du quatrième plateau à 140 1/h. On obtient la fraction de Mn valant 400 g/mole avec un réglage des paramètres chromatographiques fixant le rendement à 30 % (le rendement s'entendant ici de la proportion de matière sèche extraite de cette fraction de haut poids moléculaire par rapport à la matière sèche introduite dans le système chromatographique).

Les résultats d'analyse de cette fraction de bas poids moléculaire (produit (A)), après chromatographie, sont regroupés dans le tableau I suivant.

Tableau I

|  | Produit (A) |
| --- | --- |
| DE | 30 |
| Mn (g/mole) | 400 |
| Mw (g/mole) | 1250 |
| Indice de polymolécularité (Mw/Mn) | 3,1 |
| DP1 + DP2 (%) | 35 |
| Liaisons 1,2 (%) | 11 |
| Liaisons 1,3 (%) | 12 |
| Liaisons 1,4 (%) | 44 |
| Liaisons 1,6 (%) | 33 |
| Teneurs en fibres AOAC (%/sec) | 70,4 |

On procède à l'exclusion des molécules de DP1 et DP2 en passant cette fraction de bas poids moléculaire sur colonne de chromatographie UBR 35 K sous forme Na+.

Le rendement massique est estimé à 50 %.

Le produit ainsi obtenu est déminéralisé sur résines cationiques (C150 de PUROLITE) et anionique (Amberlite IRA 910 de ROHM & HAAS), puis éventuellement atomisé.

Les résultats d'analyse de cette maltodextrine hyperbranchée conforme à l'invention (produit (B)) sont regroupés dans le tableau II suivant.

Tableau II

|  | Produit (B) |
|---|---|
| DE | 9 |
| Mn (g/mole) | 1595 |
| Mw (g/mole) | 2715 |
| Indice de polymolécularité (Mw/Mn) | 1,7 |
| DP1 + DP2 (%) | < 0,5 |
| Liaisons 1,2 (%) | 10,3 |
| Liaisons 1,3 (%) | 9,4 |
| Liaisons 1,4 (%) | 49,6 |
| Liaisons 1,6 (%) | 30,7 |
| Teneurs en fibres AOAC (%/sec) | 78 |

On procède également à l'exclusion des molécules de DP1 et de DP2 sur le produit (A) traité préalablement à l'amyloglucosidase (OPTIDEX® L300A de GENENCOR ; 0,5 % sur sec, pH 4,5, à 60°C pendant 8 heures).

Les résultats d'analyse de cette maltodextrine hyperbranchée conforme à l'invention (produit (C)) sont regroupés dans le tableau III suivant.

Tableau III

|  | Produit (C) |
|---|---|
| DE | 14 |
| Mn (g/mole) | 865 |
| Mw (g/mole) | 2090 |
| Indice de polymolécularité (Mw/Mn) | 2, 4 |
| DP1 + DP2 (%) | 5, 3 |
| Liaisons 1,2 (%) | 9, 2 |
| Liaisons 1,3 (%) | 10, 4 |
| Liaisons 1,4 (%) | 37, 6 |
| Liaisons 1,6 (%) | 42, 8 |
| Teneurs en fibres AOAC (%/sec) | 91, 4 |

Le traitement à l'amyloglucosidase suivi d'une exclusion des DP1 et DP2 permet ainsi d'obtenir les maltodextrines hyperbranchées avec une teneur en fibres AOAC renforcée.

**Exemple 2** : **Mesure de rôle hypoglycémiant des maltodextrines hyperbranchées de l'invention**

L'évaluation *in vitro* de l'effet inhibiteur d'$\alpha$ amylase des maltodextrines standard et in situ de l'effet inhibiteur sur la digestion intestinale des maltodextrines standard a donc été réalisée sur les deux produits préparés selon les procédés décrits dans l'exemple 1.

Le résultat du facteur d'inhibition de l'activité $\alpha$-amylase pancréatique de porc sur de la GLUCIDEX® 6, en présence des maltodextrines hyperbranchées (B) et (C) de l'exemple 1, est présenté dans le tableau suivant.

Une maltodextine branchée conforme à celles préparées selon l'enseignement du brevet EP 1.006.128 de la société Demanderesse (commercialisé par la société Demanderesse sous le nom de marque NUTRIOSE® FB10), ainsi que des produits commerciaux sont également testées en Témoins.

A titre d'information, le NUTRIOSE® FB10 présente les caractéristiques suivantes :

- DE : 10
- Mw : 3996 daltons

- teneur en liaisons glucosidiques 1 → 6 : 33 %

Quant au polydextrose (commercialisé sous le nom de marque LITESSE®), il présente :

- un DE : 8
- un Mw : 1700 daltons
- une teneur en liaisons glucosidiques 1 → 6 : 42 %

Tableau IV

| Produit testé | facteur d'inhibition de l'activité $\alpha$-amylace sur la GLUCIDEX® 6 |
|---|---|
| Produit (B) | 91,9 % |
| Produit (C) | 81,1 % |
| NUTRIOSE® FB 10 | 49,6 % |
| LITESSE® | 29,4 % |

Quant à la mesure de l'inhibition de la digestion intestinale, réalisée conformément au test présenté ci-avant, elle est réalisée sur le GLUCIDEX®6 à 10 g/l, le produit (B) à 10 g/l et le produit (C) testé à 10 g/l.

Les résultats de % d'hydrolyse obtenu au cours du temps sont présentés dans le tableau suivant.

Tableau VI

| | 30 minutes | 60 minutes | 120 minutes |
|---|---|---|---|
| GLUCIDEX®6 | 59,7 $\pm$ 9,3 | 87,9 $\pm$ 5,8 | 98,0 $\pm$ 1,8 |
| Produit (B) | 20,7 $\pm$ 3,6 * | 27,7 $\pm$ 6,2 * | 33,8 $\pm$ 6,8 * |
| GLUCIDEX®6 + Produit (B) | 30,6 $\pm$ 9,1 * | 51,0 $\pm$ 6,4 * | 63,9 $\pm$ 3,6 * |
| * : $p < 0,001$ par rapport à la GLUCIDEX®6 | | | |

Le Produit (B) permet de limiter l'hydrolyse de la GLUCIDEX®6. En fin de perfusion intestinale, le pourcentage d'hydrolyse de la GLUCIDEX®6 est de 63,9 % par rapport aux 98,0 % obtenus lorsque la GLUCIDEX®6 est testée seule.

Tableau VII

| | 30 minutes | 60 minutes | 120 minutes |
|---|---|---|---|
| GLUCIDEX®6 | 59,7 $\pm$ 5,3 | 84,9 $\pm$ 4,4 | 100,5 $\pm$ 1,3 |
| Produit (C) | 12,4 $\pm$ 9,4 * | 13,7 $\pm$ 4,0 * | 20,5 $\pm$ 7,4 * |
| GLUCIDEX®6 + Produit (C) | 25,6 $\pm$ 8,0 * | 41,8 $\pm$ 4,5 * | 56,9 $\pm$ 5,8 * |
| * : $p < 0,001$ par rapport à la GLUCIDEX®6 | | | |

Le produit (C) permet de limiter l'hydrolyse de la GLUCIDEX®6. En fin de perfusion intestinale, le pourcentage d'hydrolyse de la GLUCIDEX®6 est de 56,9 % par rapport aux 100,5 % obtenus lorsque la GLUCIDEX®6 est testée seule.

**Revendications**

**1.** Maltodextrines hyperbranchées, présentant un dextrose équivalent (DE) compris entre au moins 8 et au plus 15 et un poids moléculaire ou Mw compris entre au moins 1700 et au plus 3000 daltons, **caractérisées en ce qu'**elles présentent :

- une teneur en liaisons glucosidiques 1 → 6 comprise entre au moins 30 et au plus 45 %,

- une teneur en fibres indigestibles solubles, déterminée selon la méthode AOAC N°2001-03, comprise entre au moins 75 et au plus 100 %, et
- une capacité hypoglycémiante, exprimée selon un test A, se traduisant :

   - *in vitro,* par la réduction de 80 à 90 % de l'hydrolyse par l'α-amylase de maltodextrines produites par hydrolyse acide ou enzymatique d'amidon de céréales ou de tubercules, dites « maltodextrines standard », et

   - *in situ,* par la réduction de 30 à 45 % de l'activité digestive intestinale de maltodextrines standard.

**2.** Maltodextrines hyperbranchées selon la revendication 1, présentant un DE compris entre au moins 8 et au plus 12 et un Mw compris entre au moins 2500 et au plus 3000 daltons, **caractérisée par** :

   - une teneur en liaisons glucosidiques 1 → 6 comprise entre au moins 30 et au plus 35 %,
   - une teneur en fibres indigestibles solubles, déterminée selon la méthode AOAC N°2001-03, comprise entre au moins 75 et au plus 80 %.

**3.** Maltodextrines hyperbranchées selon la revendication 1, présentant un DE compris entre au moins 12 et au plus 15 et un Mw compris entre au moins 1700 et au plus 2500 daltons, **caractérisée par** :

   - une teneur en liaisons glucosidiques 1 → 6 comprise entre au moins 35 % et au plus 45 %,
   - une teneur en fibres indigestibles solubles, déterminée selon la méthode AOAC N°2001-03, comprise entre au moins 80 et au plus 100 %.

**4.** Procédé de préparation des maltodextrines hyperbranchées selon l'une quelconque des revendications 1 à 3, **caractérisé en ce que** l'on :

   a. prépare un amidon acidifié déshydraté présentant une humidité inférieure à 5 %, de préférence inférieure ou égale à 4 %,
   b. traite l'amidon acidifié ainsi déshydraté dans un réacteur de type à couche mince à une température comprise entre 120 et 300°C, de préférence comprise entre 150 et 200°C,
   c. recueille, purifie et de préférence concentre les dérivés d'amidon branchés ainsi obtenus,
   d. effectue un fractionnement moléculaire desdits dérivés d'amidon branchés de manière à obtenir une fraction présentant :

      i. un Mn compris entre 250 et 400 g/mole,
      ii. un indice de polymolécularité compris entre 2 et 3,
      iii. une teneur en sucres réducteurs comprise entre 20 et 30 %,
      iv. un taux de liaisons glucosidiques 1 → 6 compris entre 30 et 35 %,

   e. éventuellement, traite la fraction de bas poids moléculaire ainsi obtenue par une amyloglucosidase,
   f. traite la solution ainsi obtenue sur colonne chromatographique de manière à exclure les oligomères de degré de polymérisation 1 et 2,
   g. récupère les maltodextrines hyperbranchées ainsi obtenues.

**5.** Utilisation des maltodextrines hyperbranchées selon l'une quelconque des revendications 1 à 3, ou obtenues selon le procédé de la revendication 4, pour la préparation de produits alimentaires ou pharmaceutiques.

## Patentansprüche

**1.** Hyperverzweigte Maltodextrine mit einem Dextrose-Äquivalent (DE) zwischen mindestens 8 und höchstens 15 und einem Molekulargewicht oder Mw zwischen mindestens 1700 und höchstens 3000 Dalton, **dadurch gekennzeichnet, dass** sie aufweisen:

   - einen Gehalt an 1→6-glykosidischen Bindungen zwischen mindestens 30 und höchstens 45 %,
   - einen Gehalt an löslichen unverdaulichen Fasern, bestimmt nach der AOAC-Methode Nr. 2001-03, zwischen mindestens 75 bis höchstens 100 %, und

- ein hypoglykämisches Potenzial, ausgedrückt gemäß einem Test A, das sich darstellt:

  - *in vitro,* als 80 bis 90 %ige Verringerung der Hydrolyse durch $\alpha$-Amylase von Maltodextrinen, die durch saure oder enzymatische Hydrolyse von Getreide- oder Knollenstärke hergestellt werden, sogenannte "Standard-Maltodextrine", und
  - *in situ,* als 30 bis 45 %ige Verringerung der intestinalen Verdauungsaktivität von Standard-Maltodextrinen.

2. Hyperverzweigte Maltodextrine nach Anspruch 1, die ein DE zwischen mindestens 8 und höchstens 12 und ein Mw zwischen mindestens 2500 und höchstens 3000 Dalton aufweisen, **gekennzeichnet durch:**

   - einen Gehalt an 1→6-glykosidischen Bindungen zwischen mindestens 30 und höchstens 35 %,
   - einen Gehalt an löslichen unverdaulichen Fasern, bestimmt nach der AOAC-Methode Nr. 2001-03, zwischen mindestens 75 und höchstens 80 %.

3. Hyperverzweigte Maltodextrine nach Anspruch 1, die ein DE zwischen mindestens 12 und höchstens 15 und ein Mw zwischen mindestens 1700 und höchstens 2500 Dalton aufweisen, **gekennzeichnet durch:**

   - einen Gehalt an 1→6-glykosidischen Bindungen zwischen mindestens 35 und höchstens 45 %,
   - einen Gehalt an löslichen unverdaulichen Fasern, bestimmt nach der AOAC-Methode Nr. 2001-03, zwischen mindestens 80 und höchstens 100 %.

4. Verfahren zur Herstellung der hyperverzweigten Maltodextrine nach einem der Ansprüche 1 bis 3, **dadurch gekennzeichnet, dass:**

   a. eine entwässerte, angesäuerte Stärke mit einer Feuchtigkeit von weniger als 5 %, vorzugsweise weniger als oder gleich 4 %, hergestellt wird,
   b. die so entwässerte, angesäuerte Stärke in einem Dünnschichtreaktor bei einer Temperatur zwischen 120 und 300 °C, vorzugsweise zwischen 150 und 200 °C behandelt wird,
   c. die so erhaltenen verzweigten Stärkederivate aufgefangen, gereinigt und vorzugsweise konzentriert werden,
   d. eine molekulare Fraktionierung der verzweigten Stärkederivate durchgeführt wird, sodass eine Fraktion erhalten wird, die aufweist:

      i. ein Mn zwischen 250 und 400 g/mol,
      ii. einen Polydispersitätsindex zwischen 2 und 3,
      iii. einen Gehalt an reduzierenden Zuckern zwischen 20 und 30 %,
      iv. einen Anteil an 1→6-glykosidischen Bindungen zwischen 30 und 35 %,

   e. gegebenenfalls die so erhaltene niedermolekulare Fraktion mit einer Amyloglukosidase behandelt wird,
   f. die so erhaltene Lösung auf einer chromatographischen Säule behandelt wird, sodass die Oligomere mit einem Polymerisationsgrad 1 und 2 ausgeschlossen werden,
   g. die so erhaltenen hyperverzweigten Maltodextrine gewonnen werden.

5. Verwendung der hyperverzweigten Maltodextrine nach einem der Ansprüche 1 bis 3, oder erhalten nach dem Verfahren des Anspruchs 4, für die Herstellung von Nahrungsmitteln oder pharmazeutischen Produkten.

## Claims

1. Hyperbranched maltodextrins having a dextrose equivalent (DE) of between at least 8 and at most 15 and a molecular weight or Mw of between at least 1700 and at most 3000 daltons, **characterized in that** they have:

   - a 1 → 6 glucosidic linkage content of between at least 30% and at most 45%,
   - a soluble indigestible fibers content, determined according to AOAC method No. 2001-03, of between at least 75% and at most 100%, and
   - a hypoglycemic capacity, expressed according to a test A, resulting in:

     - *in vitro,* the reduction by 80% to 90% of the $\alpha$-amylase hydrolysis of maltodextrins produced by acid hydrolysis or enzymatic hydrolysis of cereal starch or tuberous plant starch, known as "standard maltodextrins", and

- *in situ,* the reduction by 30% to 45% of the intestinal digestive activity of standard maltodextrins.

2. Hyperbranched maltodextrins according to claim 1, having a DE of between at least 8 and at most 12 and a Mw of between at least 2500 and at most 3000 daltons, **characterized by:**

- a 1 → 6 glucosidic linkage content of between at least 30% and at most 35%, and
- a soluble indigestible fibers content, determined according to AOAC method No. 2001-03, of between at least 75% and at most 80%.

3. Hyperbranched maltodextrins according to claim 1, having a DE of between at least 12 and at most 15 and a Mw of between at least 1700 and at most 2500 daltons, **characterized by:**

- a 1 → 6 glucosidic linkage content of between at least 35% and at most 45%, and
- a soluble indigestible fibers content, determined according to AOAC method No. 2001-03, of between at least 80% and at most 100%.

4. Process for preparing hyperbranched maltodextrins according to any of claims 1 to 3, **characterized by:**

a. preparing a dehydrated acidified starch having a moisture content of less than 5%, preferably less than or equal to 4%,
b. treating the thus dehydrated acidified starch in a thin-film reactor at a temperature of between 120°C, and 300°C, preferably of between 150°C and 200°C,
c. collecting, purifying, and preferably concentrating the thus obtained branched derivatives of starch,
d. performing molecular fractionation of said branched derivatives of starch so as to obtain a fraction having:

i. a Mn of between 250 and 400 g/mol,
ii. a polymolecularity index of between 2 and 3,
iii. a reducing-sugar content of between 20% and 30%, and
iv. a 1 → 6 glucosidic linkage content of between 30% and 35%,

e. optionally treating the thus obtained low-molecular-weight fraction with an amyloglucosidase,
f. treating the thus obtained solution in a chromatographic column so as to exclude the oligomers with polymerization degrees of 1 and 2,
g. recovering the thus obtained hyperbranched maltodextrins.

5. Use of the hyperbranched maltodextrins according to any of claims 1 to 3, or obtained according to the process of claim 4, for the preparation of food products or pharmaceutical products.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 368451 A
- US 5264568 A
- EP 530111 A
- JP 04237497 B
- JP 04136495 B
- EP 540421 A
- EP 535627 A
- EP 1006128 A
- EP 443789 A

**Littérature non-brevet citée dans la description**

- **ENGLYST** ; **CUMMINGS**. *American Journal of Clinical Nutrition*, 1987, vol. 45, 423-431
- **LIVESEY, G** ; **TAGAMI, H**. *Am. J. Clin. Nutr.*, 2009, vol. 89, 114-25
- **LANE-EYNON**. Determination of reducing sugars by means of Fehling's solution with methylene blue as internal indicator. *J. Soc. Chem. Ind. Trans.*, 1923, 32-36
- **HAKOMORI, S.** *J. Biol. Chem*, 1964, vol. 55, 205
- **GABRIEL BERTRAND**. *Bulletin des sciences pharmacologiques*, 01 January 1907